# EUROPEAN PATENT APPLICATION

(11) **EP 4 802 993 A1**
(43) Date of publication of application: **09.09.2026**
(21) Application number: 26161584.3
(22) Date of filing: 02.03.2026
(51) Int. Cl.: A61B 5/00, A61B 5/145, A61B 5/1455

(54) **DETERMINING THE LEVEL OF INFLAMMATION OF A SUBJECT BASED ON A DERIVED CONCENTRATION OF A PTERIN AND/OR A PTERIN DERIVATIVE WITHIN THE SUBJECT'S BODILY EMISSION**

(30) Priority: 05.03.2025 US 202563767073 P
(71) Applicant: Outsense Diagnostics Ltd., Tel-Aviv 4791011 (IL)
(72) Inventor: KAPP-BARNEA, Yaara, 4480500 Nirit (IL)
(74) Representative: Boult Wade Tennant LLP

(57) **Abstract**

Apparatus and methods are described including one or more sensors that are coupled to a toilet bowl (23), and that are configured to detect one or more parameters relating to a subject's bodily emission. A computer processor (28) receives the one or more parameters relating to the subject's bodily emission from the one or more sensors (24), derives a concentration of a pterin and/or a pterin derivative within the subject's bodily emission, and determines a level of inflammation of the subject, at least partially in response thereto. Other applications are also described.

## Description

### CROSS-REFERENCE TO RELATED APPLICATIONS

The present application claims priority from U.S. Provisional Patent Application No. 63/767,073 to Kapp-Barnea, filed March 05, 2025, entitled "Analysis of Bodily Emissions," which is incorporated herein by reference.

### TECHNICAL FIELD

Some applications of the present disclosure generally relate to analysis of bodily emissions. Specifically, some applications of the present disclosure relate to apparatus and methods for detecting and classifying biomarkers in a bodily emission of a subject.

### BACKGROUND

There are substances that may be found in a human bodily emissions that are indicative of certain conditions. For example, a subject's urine or fecal matter may contain one or more physical properties, chemical compounds, and/or microscopic components indicative of an underlying condition.

Pterins are a family of metabolites containing a bicyclic nitrogenous ring system from the precursor guanosine triphosphate (GTP). Derivatives of the parent compound, pteridine, includes small substituents such as neopterin and biopterin which can be present in urine and are termed "unconjugated pteridines." Derivatives with larger residues, e.g., folic acid, riboflavin and methanopterin, are named "conjugated pteridines."

Several biological functions of unconjugated pteridines are known. For example, biopterin functions as a cofactor for mammalian aromatic amino acid monooxygenases, and arginine conversion. Aromatic amino acid monooxygenases are involved in hydroxylation of phenylalanine, tyrosine and tryptophan. Such substances thereby control biosynthesis of the neurotransmitters including dopamine, norepinephrine and serotonin, as well as affecting phenylketonuria.

In healthy human subjects, urinary neopterin concentrations undergo a diurnal rhythm, for example, the concentration of pterin per mole of creatinine varies within a certain range (of between approximately 100 and 175 micromoles of pterin per mole of creatinine). Neopterin is produced by activated macrophages and is considered a pro-inflammatory and atherosclerotic agent. For example, immune system activation and further high level of pteridine secretion has been observed during endurance exercise infection, autoimmune processes, muscular dystrophies and neurodegenerative processes, and in malignancies.

### SUMMARY

In accordance with some applications of the present disclosure, a sensor module is disposed inside a toilet bowl and includes one or more sensors that are configured to detect one or more parameters relating to a subject's bodily emission, e.g., one or more urine-related parameters and/or one or more feces-related parameters. For some applications, the one or more sensors detect at least some of the aforementioned parameters while the bodily emission is being emitted by the subject into the toilet bowl. Alternatively or additionally, the one or more sensors detect at least some of the aforementioned parameters when the bodily emission is disposed within the toilet bowl, subsequent to its emission by the subject. For some applications, similar analysis is applied to one or more other bodily emissions of the subject, such as the subject's saliva sweat, nasal mucus, mucus, phlegm, vaginal fluid, and/or tears, *mutatis mutandis.*

For some applications, a concentration of urinary neopterin and/or a concentration of urinary biopterin is derived and is used as a diagnostic and/or a prognostic biomarker, as described in further detail hereinbelow. For some applications, a concentration of urine neopterin and/or a concentration of urinary biopterin is derived and is used as marker for monitoring a subject's health status, for screening a subject, and/or for generating a training program for the subject (which may include guidance of exercise, sleep, and/or nutrition).

For some applications, a computer processor receives one or more urine-related parameters and/or feces-related parameters of a subject (e.g., a subject suffering from a chronic inflammatory disease), and derives a concentration of one or more types of pterins within the subject's urine and/or feces. For example, the computer processor may derive the absolute concentration of prostaglandin E-major urinary metabolite (PGE-MUM) and/or neopterin, and/or a normalized concentration of prostaglandin E-major urinary metabolite (PGE-MUM) and/or neopterin (for example, the concentration of prostaglandin E-major urinary metabolite (PGE-MUM) and/or neopterin normalized with respect to creatinine concentration and/or with respect to urinary specific gravity). For some applications, the concentrations of prostaglandin E-major urinary metabolite (PGE-MUM) and/or neopterin within the subject's urine and/or feces are derived using techniques described hereinbelow.

For some applications, a computer processor determines a level of inflammation of the subject, at least partially in response to a concentration of a pterin and/or a pterin derivative within the subject's bodily emission (e.g., the subject's urine and/or feces). For some applications, the computer processor determines a level of systemic inflammation of the subject, at least partially in response to the concentration of a pterin and/or a pterin derivative within the subject's bodily emission. For some applications, the computer processor monitors progression of the subject's chronic inflammatory disease, at least partially in response to the concentration of a pterin and/or a pterin derivative within the subject's bodily emission.

For some applications, a computer processor receives one or more urine-related parameters from the one or more sensors. For some applications, at least partially based upon the one or more urine-related parameters, the computer processor determines that the subject has elevated creatinine, e.g., by deriving urinary creatinine concentration using techniques described hereinbelow. For some applications, the computer processor derives a concentration of one or more additional entities within the subject's urine, and determines that a likely cause of the elevated creatinine concentration is catabolic processes at least partially in response to deriving the concentration of the one or more additional entities within the subject's urine. For some such applications, the one or more additional entities include neopterin and/or biopterin. Alternatively or additionally, the one or more additional entities include myoglobin. For some applications, the one or more additional entities include NT-Titin. For some applications, the concentrations of the one or more additional entities are derived using techniques described hereinbelow.

For some applications, the computer processor derives a concentration of one or more entities within the subject's urine that are indicative of catabolic processes (for example, neopterin, biopterin, myoglobin, and/or NT-titin, or the concentration of creatinine in addition to the concentration of neopterin, biopterin, myoglobin, and/or NT-titin). For some applications, the concentrations of the one or more entities within the subject's urine that are indicative of catabolic processes are derived using techniques described hereinbelow. In some applications, the computer processor determines strain-recovery interval for the subject, at least partially in response thereto.

For some applications, the computer processor periodically receives the one or more urine-related parameters from the one or more sensors, and determines strain-recovery intervals for the subject, at least partially in response thereto. The computer processor dynamically develops a training schedule for the subject based upon the subject's strain-recovery intervals. For example, the computer processor may generate outputs that provide guidance to the subject of the subject's exercise, sleep, and/or nutrition.

For some applications, the computer processor receives the one or more urine-related parameters from the one or more sensors, and derive a concentration of one or more types of pterins within the subject's urine. For example, the computer processor may derive the absolute concentration of neopterin and/or biopterin, and/or a normalized concentration of neopterin and/or biopterin (for example, the concentration of neopterin and/or biopterin normalized with respect to creatinine concentration and/or with respect to urinary specific gravity). For some applications, the concentrations of the one or more types of pterins within the subject's urine are derived using techniques described hereinbelow.

For some applications, the computer processor detects a neurodegenerative process of the subject, at least partially in response to a concentration of a pterin and/or a pterin derivative within a subject's urine.

There is therefor provided, in accordance with some embodiments of the present disclosure, the following examples, at least some of which are independent aspects of the present disclosure.

According to an independent aspect, the present disclosure includes example 1:
Example 1. An apparatus for use with a bodily emission of the subject, the apparatus comprising:
   one or more sensors that are coupled to a toilet bowl, and that are configured to detect one or more parameters relating to the subject's bodily emission; and
   at least one computer processor configured to:
      receive the one or more parameters relating to the subject's bodily emission from the one or more sensors,
      derive a concentration of a pterin and/or a pterin derivative within the subject's bodily emission, and
      determine a level of inflammation of the subject, at least partially in response thereto.
Example 2. The apparatus according to example 1, wherein the at least one computer processor is configured to determine a level of systemic inflammation of the subject, at least partially in response to the concentration of the pterin and/or the pterin derivative within the subject's bodily emission.
Example 3. The apparatus according to example 1, wherein the at least one computer processor is configured to monitor progression of chronic inflammatory disease of the subject, at least partially in response to the concentration of the pterin and/or the pterin derivative within the subject's bodily emission.
Example 4. The apparatus according to example 1, wherein the bodily emission includes urine and wherein the computer processor is configured to derive the concentration of the pterin and/or the pterin derivative within the subject's bodily emission by deriving a concentration of prostaglandin E-major urinary metabolite within the urine.
Example 5. The apparatus according to example 1, wherein the bodily emission includes urine and wherein the computer processor is configured to derive the concentration of the pterin and/or the pterin derivative within the subject's bodily emission by deriving a concentration of neopterin within the urine.
Example 6. The apparatus according to example 1, wherein the bodily emission includes feces and wherein the computer processor is configured to derive the concentration of the pterin and/or the pterin derivative within the subject's bodily emission by deriving a concentration of neopterin within the feces.
Example 7. The apparatus according to any one of examples 1-6, wherein the one or more sensors comprise one or more light sensors that are configured to detect light in a range of between 200 nm and 400 nm, and wherein the computer processor is configured to derive the concentration of the pterin and/or the pterin derivative within the subject's bodily emission by detecting an optical signature of the pterin and/or the pterin derivative within the detected light.
Example 8. The apparatus according to example 7, wherein the computer processor is configured to detect the optical signature of the pterin and/or the pterin derivative in its acidic form within the detected light by detecting light intensity that is indicative of one or more absorbance characteristics selected from the group consisting of:
   an absorbance trough that is centered between 240 and 265 nm,
   an absorbance peak centered between 265 and 285 nm,
   an absorbance trough that is centered between 285 and 310 nm, and
   an absorbance peak centered between 330 and 360 nm.
Example 9. The apparatus according to example 8, wherein the computer processor is configured to detect the optical signature of the pterin and/or the pterin derivative in its acidic form within the detected light by detecting a ratio between light intensity at at least one of one of the absorbance troughs and light intensity at at least one of the absorbance peaks.
Example 10. The apparatus according to example 7, wherein the computer processor is configured to detect the optical signature of the pterin and/or the pterin derivative in its basic form within the detected light by detecting light intensity that is indicative of one or more absorbance characteristics selected from the group consisting of:
   an absorbance trough that is centered between 220 and 240 nm,
   an absorbance peak centered between 245 and 265 nm,
   an absorbance trough that is centered between 290 and 310 nm, and
   an absorbance peak centered between 350 and 370 nm.
Example 11. The apparatus according to example 10, wherein the computer processor is configured to detect the optical signature of the pterin and/or the pterin derivative in its basic form within the detected light by detecting a ratio between light intensity at at least one of one of the absorbance troughs and light intensity at at least one of the absorbance peaks.

According to an independent aspect, the present disclosure includes example 12:
Example 12. A method for use with a bodily emission of the subject, the method comprising:
   using at least one computer processor:
   receiving the one or more parameters relating to the subject's bodily emission from one or more light sensors that are coupled to a toilet bowl,
   deriving a concentration of a pterin and/or a pterin derivative within the subject's bodily emission, and
   determining a level of inflammation of the subject, at least partially in response thereto.
Example 13. The method according to example 12, wherein determining the level of inflammation of the subject at least partially in response in response to the concentration of the pterin and/or the pterin derivative within the subject's bodily emission comprises determining a level of systemic inflammation of the subject, at least partially in response to the concentration of the pterin and/or the pterin derivative within the subject's bodily emission.
Example 14. The method according to example 12, wherein determining the level of inflammation of the subject at least partially in response in response to the concentration of the pterin and/or the pterin derivative within the subject's bodily emission comprises monitoring progression of chronic inflammatory disease of the subject, at least partially in response to the concentration of the pterin and/or the pterin derivative within the subject's bodily emission.
Example 15. The method according to example 12, wherein the bodily emission includes urine, and wherein deriving the concentration of the pterin and/or the pterin derivative within the subject's bodily emission comprises deriving a concentration of prostaglandin E-major urinary metabolite within the urine.
Example 16. The method according to example 12, wherein the bodily emission includes urine, and wherein deriving the concentration of the pterin and/or the pterin derivative within the subject's bodily emission comprises deriving a concentration of neopterin within the urine.
Example 17. The method according to example 12, wherein the bodily emission includes feces, and wherein deriving the concentration of the pterin and/or the pterin derivative within the subject's bodily emission comprises deriving a concentration of neopterin within the feces.
Example 18. The method according to any one of examples 12-17, wherein the one or more light sensors are configured to detect light in a range of between 200 nm and 400 nm, and wherein deriving the concentration of the pterin and/or the pterin derivative within the subject's bodily emission comprises detecting an optical signature of the pterin and/or the pterin derivative within the detected light.
Example 19. The method according to example 18, wherein detecting the optical signature of the pterin and/or the pterin derivative within the detected light comprises detecting the optical signature of the pterin and/or the pterin derivative in its acidic form within the detected light by detecting light intensity that is indicative of one or more absorbance characteristics selected from the group consisting of:
   an absorbance trough that is centered between 240 and 265 nm,
   an absorbance peak centered between 265 and 285 nm,
   an absorbance trough that is centered between 285 and 310 nm, and
   an absorbance peak centered between 330 and 360 nm.
Example 20. The method according to example 19, wherein detecting the optical signature of the pterin and/or the pterin derivative in its acidic form within the detected light comprises detecting a ratio between light intensity at at least one of one of the absorbance troughs and light intensity at at least one of the absorbance peaks.
Example 21. The method according to example 18, wherein detecting the optical signature of the pterin and/or the pterin derivative within the detected light comprises detecting the optical signature of the pterin and/or the pterin derivative in its basic form within the detected light by detecting light intensity that is indicative of one or more absorbance characteristics selected from the group consisting of:
   an absorbance trough that is centered between 220 and 240 nm,
   an absorbance peak centered between 245 and 265 nm,
   an absorbance trough that is centered between 290 and 310 nm, and
   an absorbance peak centered between 350 and 370 nm.
Example 22. The method according to example 21, wherein detecting the optical signature of the pterin and/or the pterin derivative in its basic form within the detected light comprises detecting a ratio between light intensity at at least one of one of the absorbance troughs and light intensity at at least one of the absorbance peaks.

According to an independent aspect, the present disclosure includes example 23:
Example 23. An apparatus comprising:
   one or more sensors that are coupled to a toilet bowl, and that are configured to detect one or more urine-related parameters relating to a subject's urine; and
   at least one computer processor configured to:
      periodically, receive the one or more urine-related parameters from the one or more sensors,
      determine strain-recovery intervals for the subject, at least partially in response thereto; and
      dynamically develop a training schedule for the subject based upon the subject's strain-recovery intervals.
Example 24. The apparatus according to example 23, wherein the at least one computer processor is configured to periodically receive the one or more urine-related parameters from the one or more sensors by periodically receiving one or more urine-related parameters from the one or more sensors that are indicative of a concentration of myoglobin within the subject's urine, and the at least one computer processor is configured to derive a urinary myoglobin concentration therefrom.
Example 25. The apparatus according to example 23, wherein the at least one computer processor is configured to periodically receive the one or more urine-related parameters from the one or more sensors by periodically receiving one or more urine-related parameters from the one or more sensors that are indicative of a concentration of NT-titin within the subject's urine, and the at least one computer processor is configured to derive a urinary NT-titin concentration therefrom.
Example 26. The apparatus according to example 23, wherein the at least one computer processor is configured to generate an output that provides guidance to the subject with respect to scheduling of an activity selected from the group consisting of: exercise, sleep, mindfulness, a stress-relieving activity, and nutritional intake.
Example 27. The apparatus according to any one of examples 23-26, wherein the at least one computer processor is configured to receive inputs that are indicative of an activity of the subject selected from the group consisting of: exercise, sleep, mindfulness, a stress-relieving activity, and nutritional intake, and wherein the computer processor is configured to dynamically develop the training schedule for the subject based upon the subject's strain-recovery intervals and the received inputs.
Example 28. The apparatus according to example 27, wherein the computer processor is configured to receive the input from the subject.
Example 29. The apparatus according to example 27, wherein the computer processor is configured for use with a device that is configured to automatically monitor the selected activity and wherein the computer processor is configured to receive the input from the device.
Example 30. The apparatus according to example 23, wherein the at least one computer processor is configured to periodically receive the one or more urine-related parameters from the one or more sensors by periodically receiving one or more urine-related parameters from the one or more sensors that are indicative of a concentration of a pterin and/or a pterin derivative within the subject's urine, and the at least one computer processor is configured to derive the concentration of the pterin and/or the pterin derivative from the one or more urine-related parameters.
Example 31. The apparatus according to example 30, wherein the at least one computer processor is configured to periodically receive the one or more urine-related parameters from the one or more sensors by periodically receiving one or more urine-related parameters from the one or more sensors that are indicative of a concentration of neopterin within the subject's urine, and the at least one computer processor is configured to derive a urinary neopterin concentration therefrom.
Example 32. The apparatus according to example 30, wherein the at least one computer processor is configured to periodically receive the one or more urine-related parameters from the one or more sensors by periodically receiving one or more urine-related parameters from the one or more sensors that are indicative of a concentration of biopterin within the subject's urine, and the at least one computer processor is configured to derive a urinary biopterin concentration therefrom.
Example 33. The apparatus according to example 30, wherein the one or more sensors comprise one or more light sensors that are configured to detect light in a range of between 200 nm and 400 nm, and wherein the computer processor is configured to derive the concentration of the pterin and/or the pterin derivative within the subject's urine by detecting an optical signature of the pterin and/or the pterin derivative within the detected light.
Example 34. The apparatus according to example 33, wherein the computer processor is configured to detect the optical signature of the pterin and/or the pterin derivative in its acidic form within the detected light by detecting light intensity that is indicative of one or more absorbance characteristics selected from the group consisting of:
   an absorbance trough that is centered between 240 and 265 nm,
   an absorbance peak centered between 265 and 285 nm,
   an absorbance trough that is centered between 285 and 310 nm, and
   an absorbance peak centered between 330 and 360 nm.
Example 35. The apparatus according to example 34, wherein the computer processor is configured to detect the optical signature of the pterin and/or the pterin derivative in its acidic form within the detected light by detecting a ratio between light intensity at at least one of one of the absorbance troughs and light intensity at at least one of the absorbance peaks.
Example 36. The apparatus according to example 33, wherein the computer processor is configured to detect the optical signature of the pterin and/or the pterin derivative in its basic form within the detected light by detecting light intensity that is indicative of one or more absorbance characteristics selected from the group consisting of:
   an absorbance trough that is centered between 220 and 240 nm,
   an absorbance peak centered between 245 and 265 nm,
   an absorbance trough that is centered between 290 and 310 nm, and
   an absorbance peak centered between 350 and 370 nm.
Example 37. The apparatus according to example 36, wherein the computer processor is configured to detect the optical signature of the pterin and/or the pterin derivative in its basic form within the detected light by detecting a ratio between light intensity at at least one of one of the absorbance troughs and light intensity at at least one of the absorbance peaks.

According to an independent aspect, the present disclosure includes example 38:
Example 38. A method comprising:
using at least one computer processor:
periodically receiving one or more urine-related parameters relating to a subject's urine from one or more light sensors that are coupled to a toilet bowl,
determining strain-recovery intervals for the subject, at least partially in response thereto; and
dynamically developing a training schedule for the subject based upon the subject's strain-recovery intervals.

According to an independent aspect, the present disclosure includes example 39:
Example 39. An apparatus for use with urine of a subject, the apparatus comprising:
   one or more sensors that are coupled to a toilet bowl, and that are configured to detect one or more urine-related parameters relating to the subject's urine; and
   at least one computer processor configured to:
      receive the one or more urine-related parameters from the one or more sensors,
      derive a concentration of a pterin and/or a pterin derivative within the subject's urine, and
      detect a neurodegenerative process of the subject, at least partially in response thereto.
Example 40. The apparatus according to example 39, wherein the one or more sensors comprise one or more light sensors that are configured to detect light in a range of between 200 nm and 400 nm, and wherein the computer processor is configured to derive the concentration of the pterin and/or the pterin derivative within the subject's urine by detecting an optical signature of the pterin and/or the pterin derivative within the detected light.
Example 41. The apparatus according to example 40, wherein the computer processor is configured to detect the optical signature of the pterin and/or the pterin derivative in its acidic form within the detected light by detecting light intensity that is indicative of one or more absorbance characteristics selected from the group consisting of:
   an absorbance trough that is centered between 240 and 265 nm,
   an absorbance peak centered between 265 and 285 nm,
   an absorbance trough that is centered between 285 and 310 nm, and
   an absorbance peak centered between 330 and 360 nm.
Example 42. The apparatus according to example 41, wherein the computer processor is configured to detect the optical signature of the pterin and/or the pterin derivative in its acidic form within the detected light by detecting a ratio between light intensity at at least one of one of the absorbance troughs and light intensity at at least one of the absorbance peaks.
Example 43. The apparatus according to example 40, wherein the computer processor is configured to detect the optical signature of the pterin and/or the pterin derivative in its basic form within the detected light by detecting light intensity that is indicative of one or more absorbance characteristics selected from the group consisting of:
   an absorbance trough that is centered between 220 and 240 nm,
   an absorbance peak centered between 245 and 265 nm,
   an absorbance trough that is centered between 290 and 310 nm, and
   an absorbance peak centered between 350 and 370 nm.
Example 44. The apparatus according to example 43, wherein the computer processor is configured to detect the optical signature of the pterin and/or the pterin derivative in its basic form within the detected light by detecting a ratio between light intensity at at least one of one of the absorbance troughs and light intensity at at least one of the absorbance peaks.

According to an independent aspect, the present disclosure includes example 45:
Example 45. A method comprising:
   using at least one computer processor:
   receiving one or more urine-related parameters relating to a subject's urine from one or more light sensors that are coupled to a toilet bowl,
   deriving a concentration of a pterin and/or a pterin derivative within the subject's urine, and
   detecting a neurodegenerative process of the subject, at least partially in response thereto.
Example 46. An apparatus comprising:
   one or more sensors that are coupled to a toilet bowl, and that are configured to detect one or more urine-related parameters relating to a subject's urine; and
   at least one computer processor configured to:
      receive the one or more urine-related parameters from the one or more sensors,
      derive a concentration of one or more entities within the subject's urine that are indicative of catabolic processes, and
      determine a strain-recovery interval for the subject, at least partially in response thereto.
Example 47. The apparatus according to example 46, wherein the at least one computer processor is configured to derive the concentration of one or more entities within the subject's urine that are indicative of catabolic processes by deriving a concentration of myoglobin within the subject's urine.
Example 48. The apparatus according to example 46, wherein the at least one computer processor is configured to derive the concentration of one or more entities within the subject's urine that are indicative of catabolic processes by deriving a concentration of NT-titin within the subject's urine.
Example 49. The apparatus according to example 46, wherein the at least one computer processor is configured to derive the concentration of one or more entities within the subject's urine that are indicative of catabolic processes by deriving a concentration of a pterin and/or a pterin derivative within the subject's urine.
Example 50. The apparatus according to example 49, wherein the at least one computer processor is configured to derive the concentration of one or more entities within the subject's urine that are indicative of catabolic processes by deriving a concentration of neopterin within the subject's urine.
Example 51. The apparatus according to example 49, wherein the at least one computer processor is configured to derive the concentration of one or more entities within the subject's urine that are indicative of catabolic processes by deriving a concentration of biopterin.
Example 52. The apparatus according to example 49, wherein the one or more sensors comprise one or more light sensors that are configured to detect light in s range of between 200 nm and 400 nm, and wherein the computer processor is configured to detect the concentration of the pterin and/or the pterin derivative within the subject's urine by detecting an optical signature of the pterin and/or the pterin derivative within the detected light.
Example 53. The apparatus according to example 52, wherein the computer processor is configured to detect the optical signature of the pterin and/or the pterin derivative in its acidic form within the detected light by detecting light intensity that is indicative of one or more absorbance characteristics selected from the group consisting of:
   an absorbance trough that is centered between 240 and 265 nm,
   an absorbance peak centered between 265 and 285 nm,
   an absorbance trough that is centered between 285 and 310 nm, and
   an absorbance peak centered between 330 and 360 nm.
Example 54. The apparatus according to example 53, wherein the computer processor is configured to detect the optical signature of the pterin and/or the pterin derivative in its acidic form within the detected light by detecting a ratio between light intensity at at least one of one of the absorbance troughs and light intensity at at least one of the absorbance peaks.
Example 55. The apparatus according to example 52, wherein the computer processor is configured to detect the optical signature of the pterin and/or the pterin derivative in its basic form within the detected light by detecting light intensity that is indicative of one or more absorbance characteristics selected from the group consisting of:
   an absorbance trough that is centered between 220 and 240 nm,
   an absorbance peak centered between 245 and 265 nm,
   an absorbance trough that is centered between 290 and 310 nm, and
   an absorbance peak centered between 350 and 370 nm.
Example 56. The apparatus according to example 55, wherein the computer processor is configured to detect the optical signature of the pterin and/or the pterin derivative in its basic form within the detected light by detecting a ratio between light intensity at at least one of one of the absorbance troughs and light intensity at at least one of the absorbance peaks.

According to an independent aspect, the present disclosure includes example 57:
Example 57. A method comprising:
using at least one computer processor:
receiving one or more urine-related parameters relating to a subject's urine from one or more light sensors that are coupled to a toilet bowl,
deriving a concentration of one or more entities within the subject's urine that are indicative of catabolic processes, and
determining a strain-recovery interval for the subject, at least partially in response thereto.

The present disclosure will be more fully understood from the following detailed description of embodiments thereof, taken together with the drawings, in which:

### BRIEF DESCRIPTION OF THE DRAWINGS

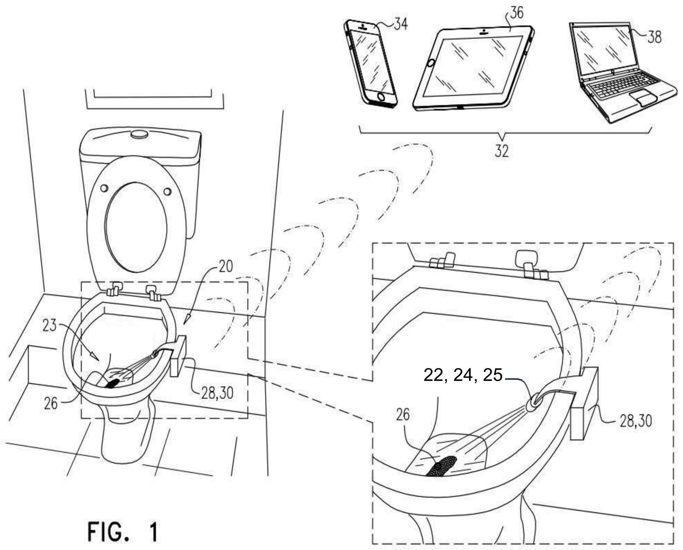

Fig. 1 is a schematic illustration of apparatus for analyzing a bodily emission, in accordance with some applications of the present disclosure.

### DETAILED DESCRIPTION

Reference is now made to Fig. 1, which is a schematic illustration of apparatus 20 for analyzing bodily emissions 26, in accordance with some applications of the present disclosure. As shown, for some applications, apparatus 20 includes a sensor module 22, which is placed inside a toilet bowl 23. For some applications (not shown), the sensor module (and/or additional components of the apparatus) is integrated into the toilet bowl. The sensor module includes one or more sensors 24. For some applications, the one or more sensors are configured to detect one or more urine-related parameters (such as, voiding frequency, urine volume, urine color, and/or urine concentration, etc.), and/or to detect one or more feces-related parameters (such as, feces shape, size, texture, etc.).

For some applications, the one or more sensors detect at least some of the aforementioned parameters while the bodily emission is being emitted by the subject into the toilet bowl. Alternatively or additionally, the one or more sensors detect at least some of the aforementioned parameters when the bodily emission is disposed within the toilet bowl, subsequent to its emission by the subject. For some applications, similar detection and analysis is applied to one or more other bodily emissions of the subject, such as the subject's saliva sweat, nasal mucus, mucus, phlegm, vaginal fluid, and/or tears, *mutatis mutandis.*

For some applications, the sensors include an imaging component, such as an RGB camera, a spectral camera, and/or a hyperspectral camera. Alternatively or additionally, the one or more sensors include one or more light sensors that are configured to receive light from the bodily emissions. For some applications, the sensor module includes one or more illumination components 25. In accordance with respective applications, such illumination components include illumination components that are configured to illuminate the bodily emissions at given spectral bands (e.g., LEDs and/or lasers), and/or a broadband light source. For some applications, a broadband light source is used in combination with one or more bandpass filters (which may be used to filter the emitted and/or the detected light).

For some applications, a computer processor receives the one or more urine-related parameters from the one or more sensors, and/or receives the one or more feces-related parameters from the one or more sensors. In accordance with respective applications, the computer processor that performs the analysis described herein is a computer processor 28 disposed inside housing 30 (which may also house the sensor module), or is a different computer processor that is in communication with the sensor module. Accordingly, although some processing steps are described hereinbelow as being performed by processor 28, the scope of the present disclosure includes using one or more additional processors to perform these steps, as an alternative to, or in addition to, processor 28.

For some applications, apparatus 20 includes a power source (e.g., a battery, a miniature battery, a capacitor, a chemical energy storage device, etc.), that is disposed outside the toilet bowl inside housing 30. Alternatively or additionally, the sensor module is connected to mains electricity (not shown). For some applications, the power source and sensor module 22 are connected wiredly, or wirelessly. In accordance with respective applications, the computer processor that performs the analysis described herein is disposed inside the toilet bowl (e.g., computer processor 28 disposed inside housing 30 (which may also house the sensor module)), or remotely. For example, as shown, the sensor module may communicate wirelessly with a user interface device 32 that includes a computer processor. Such a user interface device may include, but is not limited to, a phone 34, a tablet computer 36, a laptop computer 38, or a different sort of personal computing device. For some applications the user interface device acts as both an input device and an output device, via which the user interacts with sensor module 22. The sensor module may transmit data to the user interface device and the user interface device computer processor may run a program that is configured to analyze the received data.

For some applications, sensor module 22 and/or the user interface device 32 communicates with a remote server, e.g., to thereby communicate with a third-party device. For example, the apparatus may communicate with a physician or an insurance company, via a third-party device, over a communication network without intervention from the subject. The physician or the insurance company may evaluate the results and determine whether further testing or intervention is appropriate for the subject.

For some applications, data relating to the received sensor signals are stored in a memory. For example, the memory may be disposed inside the toilet bowel (e.g., inside the sensor unit), inside housing 30, or remotely. For some applications, memory associated within computer processor 28 stores data relating to the received signals. Periodically, the subject may submit the stored data to a third-party facility, such as a healthcare facility (e.g., a physician's office, or a pharmacy) or an insurance company, and a computer processor of the remote device at the facility may then perform the above-described analysis on a batch of data relating to a plurality of bodily emissions of the subject that were acquired over a period of time.

It is noted that, for some applications, the apparatus and methods described herein include a screening test in which the subject is not required to physically touch the bodily emission. Furthermore, for some applications, the subject is only required to touch any portion of the dedicated sensing apparatus periodically, for example, in order to install the device, or to change or recharge the device batteries. (It is noted that the subject may handle the user interface device, but this is a device (such as a phone) that the subject handles even when not using the sensing apparatus.) For some applications, the apparatus and methods described herein do not require adding anything to the toilet bowl subsequent to the subject emitting a bodily emission into the toilet bowl, in order to facilitate the analysis of the emission, and/or a determination that the subject is suffering from dehydration and the classification thereof. For some applications, the subject is not required to perform any action after installation of the apparatus in the toilet bowl. The testing is automatic and handled by the apparatus, and monitoring of the subject's emissions is seamless to the subject and does not require compliance by the subject, so long as no abnormality is detected.

For some applications, subsequent to the subject emitting a bodily emission into the toilet bowl (and optionally once the subject has finished excreting the bodily emission, and the bodily emission is at least partially disposed within the water of the toilet bowl), the bodily emission is imaged by receiving reflected and/or transmitted light from the toilet bowl, without requiring any action to be performed by any person subsequent to the emission. For some applications, the bodily emission is analyzed during the emission of the bodily emission into the toilet bowl.

For some applications, for each emission of the subject, in the case of any findings, the apparatus reports the findings to the subject via an output device, e.g., via user interface device 32. For some applications, the output device includes an output component (such as a light (e.g., an LED) or a screen) that is built into apparatus 20.

For some applications, sensor module 22 is disposed inside the toilet bowl. For some applications, the sensor module includes an imaging component, which in turn includes one or more light sensors that are configured to receive light from bodily emissions that were emitted by the subject and are disposed inside the toilet bowl. For some applications, the sensor module is housed in a water-resistant housing. For some applications, the face of the sensor module underneath which the imaging component is mounted is covered with a transparent, water-resistant cover. It is noted that Fig. 1 shows the sensor module disposed above the water level of the water within the toilet bowl. However, for some applications, at least a portion of the sensor module (e.g., the entire sensor module) is submerged within the water in the toilet bowl.

For some applications, sensor module 22 includes a subject sensor. The subject sensor is configured to detect when a subject is on or in the vicinity of the toilet, and/or if the subject has defecated and/or urinated into the toilet bowl. For example, the subject sensor may include a motion sensor, configured to sense the motion of feces, urine, the subject, or the water in the toilet bowl. Alternatively or additionally, the subject sensor may include a light sensor configured to detect when the light in the bathroom is switched on, or when the subject sits on the toilet. For some applications, light sensors that are used for detecting light from the bodily emission are also used for the aforementioned function. For some such applications, the sensor module is configured to be in standby mode most of the time (such that the sensor module uses a reduced amount of power). The sensor module is switched on in response to detecting that the subject is on or in the vicinity of the toilet, and/or that the subject has defecated and/or urinated into the toilet bowl. For some applications, the imaging and/or sensing components of the sensor module acquire data in response to detecting that the subject is on or in the vicinity of the toilet, and/or that the subject has defecated and/or urinated into the toilet bowl. For some applications, the subject switches on the sensor module manually.

For some applications, computer processor 28 receives one or more urine-related parameters from the one or more sensors. For some applications, at least partially based upon the one or more urine-related parameters, the computer processor determines that the subject has elevated creatinine, e.g., by deriving urinary creatinine concentration using techniques described hereinbelow. For some applications, the computer processor derives a concentration of one or more additional entities within the subject's urine, and determines that a likely cause of the elevated creatinine concentration is catabolic processes at least partially in response to deriving the concentration of the one or more additional entities within the subject's urine. For some such applications, the one or more additional entities include a pterin and/or a pterin derivative. For some such applications, the one or more additional entities include neopterin and/or biopterin. Alternatively or additionally, the one or more additional entities include myoglobin. For some applications, the one or more additional entities include NT-Titin. For some applications, the concentrations of the one or more additional entities are derived using techniques described hereinbelow.

For some applications, computer processor 28 derives a concentration of one or more entities within the subject's urine (for example, neopterin, biopterin, myoglobin, and/or NT-titin, or the concentration of creatinine in addition to the concentration of neopterin, biopterin, myoglobin, and/or NT-titin) that are indicative of catabolic processes, i.e., the sequences of enzyme-catalyzed reactions by which relatively large molecules in living cells are broken down, or degraded.

It is noted that, as athletes strive to improve their performance, modifications in exercise, sleep, mindfulness (and/or other stress-relieving activities) and/or nutrition are required. Under extreme exercise, the human body is pushed to its limits and damage may occur including orthopedic trauma accompanied by muscle injuries, cardiac risk, inflammatory responses, etc. During intensive exercise, the concentration of pterins and/or pterin derivatives (and/or the concentration of pterins and/or pterin derivatives when normalized with respect to creatinine) tend to increase, which is indicative of strain recovery. Even after the exercise, the concentration of pterins and/or pterin derivatives remains elevated at its peak for several days (e.g., 3-4 days) before slowly declining towards baseline levels (over a further period of 5-10 days).

For some applications, the concentrations of the one or more entities within the subject's urine that are indicative of catabolic processes are derived using techniques described hereinbelow. In some applications, the computer processor determines a strain-recovery interval for the subject, at least partially in response thereto.

For some applications, computer processor 28 periodically receives the one or more urine-related parameters from the one or more sensors, and determines strain-recovery intervals for the subject, at least partially in response thereto. The computer processor dynamically develops a training schedule for the subject based upon the subject's strain-recovery intervals. For example, the computer processor may generate outputs that provide guidance to the subject of the subject's exercise, sleep, mindfulness (and/or other stress-relieving activities) and/or nutrition.

For some applications, in addition to receiving the one or more urine-related parameters from the one or more sensors, the computer processor is configured to receive inputs from the subject or from another monitoring device (e.g., a smart watch or a smartphone) that is indicative of the subject's exercise, sleep, mindfulness (and/or other stress-relieving activities) and/or nutrition. For some applications, the computer processor is configured to identify patterns between (a) the subject's exercise, sleep, mindfulness (and/or other stress-relieving activities) and/or nutrition and (b) strain-recovery intervals for the subject. For some applications, based on the identified patterns, the computer processor generates outputs that provide guidance to the subject of the subject's exercise, sleep, mindfulness (and/or other stress-relieving activities) and/or nutrition. For some applications, the outputs are configured to reduce the subject's strain-recovery interval (as indicated by urinary pterin concentration and/or urinary pterin-derivative concentration). By dynamically monitoring urinary pterin concentration (and/or urinary pterin-derivative concentration) and the subject's exercise, sleep, mindfulness (and/or other stress-relieving activities) and/or nutrition, the computer processor is configured to identify patterns of activity that will reduce the subject's strain-recovery interval (as indicated by urinary pterin concentration and/or urinary pterin-derivative concentration).

For some applications, computer processor 28 receives the one or more urine-related parameters from the one or more sensors, and derives a concentration of one or more types of pterins and/or pterin derivatives within the subject's urine. For example, the computer processor may derive the absolute concentration of neopterin and/or biopterin, and/or a normalized concentration of neopterin and/or biopterin (for example, the concentration of neopterin and/or biopterin within the subject's urine normalized with respect to creatinine concentration and/or with respect to urinary specific gravity). Alternatively or additionally, computer processor 28 receives one or more feces-related parameters of a subject from the one or more sensors, and derives a concentration of one or more types of pterins and/or pterin derivatives within the subject's feces. For example, the computer processor may derive the absolute concentration of prostaglandin E-major urinary metabolite (PGE-MUM) and/or neopterin, and/or a normalized concentration of prostaglandin E-major urinary metabolite (PGE-MUM) and/or neopterin (for example, the concentration of prostaglandin E-major urinary metabolite (PGE-MUM) and/or neopterin normalized with respect to creatinine concentration and/or with respect to urinary specific gravity). For some applications, the concentrations of prostaglandin E-major urinary metabolite (PGE-MUM) and/or neopterin within the subject's urine and/or feces are derived using techniques described hereinbelow. For some applications, the computer processor determines a level of systemic inflammation of the subject, at least partially in response thereto.

It is noted that Serum C-Reactive Protein (CRP) is a well-established commonly used index for systemic inflammation. Using the presently described technique of deriving the concentration of one or more types of pterins within the subject's urine in order to determine a level of systemic inflammation of the subject, can be used as a screening technique for the general population (or a subpopulation having risk factors), thereby allowing for continuous noninvasive monitoring of the general population.

For some applications, computer processor 28 receives the one or more urine-related parameters from the one or more sensors, and derives a concentration of one or more types of pterins and/or pterin derivatives within the subject's urine. For example, the computer processor may derive the absolute concentration of neopterin and/or biopterin, and/or a normalized concentration of neopterin and/or biopterin (for example, the concentration of neopterin and/or biopterin within the subject's urine normalized with respect to creatinine concentration and/or with respect to urinary specific gravity). Alternatively or additionally, computer processor 28 receives one or more feces-related parameters of a subject from the one or more sensors, and derives a concentration of one or more types of pterins and/or pterin derivatives within the subject's feces. For example, the computer processor may derive the absolute concentration of prostaglandin E-major urinary metabolite (PGE-MUM) and/or neopterin, and/or a normalized concentration of prostaglandin E-major urinary metabolite (PGE-MUM) and/or neopterin (for example, the concentration of prostaglandin E-major urinary metabolite (PGE-MUM) and/or neopterin normalized with respect to creatinine concentration and/or with respect to urinary specific gravity). For some applications, the concentrations of prostaglandin E-major urinary metabolite (PGE-MUM) and/or neopterin within the subject's urine and/or feces are derived using techniques described hereinbelow. For some applications, the computer processor monitors progression of the subject's chronic inflammatory disease, at least partially in response thereto.

For some applications, the computer processor receives the one or more urine-related parameters from the one or more sensors, and derives a concentration of one or more types of pterins and/or pterin derivatives within the subject's urine. For example, the computer processor may derive the absolute concentration of neopterin and/or biopterin, and/or a normalized concentration of neopterin and/or biopterin (for example, the concentration of neopterin and/or biopterin normalized with respect to creatinine concentration and/or with respect to urinary specific gravity). For some applications, the concentrations of the one or more types of pterins and/or pterin derivatives within the subject's urine are derived using techniques described hereinbelow. For some applications, the computer processor detects a neurodegenerative process of the subject, at least partially in response thereto.

For some applications, sensors 24 within sensor module 22 include an imaging component, such as an RGB camera, a spectral camera, and/or a hyperspectral camera. Alternatively or additionally, the one or more sensors may include one or more light sensors that are configured to receive light from the bodily emissions. For some applications, the sensor module includes one or more illumination components. In accordance with respective applications, such illumination components include illumination components that are configured to illuminate the bodily emissions at given spectral bands (e.g., LEDs and/or lasers), and/or a broadband light source. For some applications, a broadband light source is used in combination with one or more bandpass filters (which may be used to filter the emitted and/or the detected light).

For some applications, the toilet bowl is illuminated with broadband light and absorption of light within a given spectral range is detected in order determine the concentration of one or more components within a subject's bodily emission. For some applications, the toilet bowl is illuminated with light within specific wavelength bands that are of interest determine the concentration of one or more components within a subject's bodily emission. For some applications, ambient light is used to illuminate the bodily emission.

For some applications, the sensors are configured to detect light in the visible range and the computer processor is configured to determine the concentration of one or more components within a subject's bodily emission, based on the light that is detected in the visible range. Alternatively or additionally, the sensors are configured to detect light in the near-infrared and/or mid-infrared range and the computer processor is configured to determine the concentration of one or more components within a subject's bodily emission, based on the light that is detected in the near-infrared and/or mid-infrared range. Further alternatively or additionally, the sensors are configured to detect light in the ultraviolet range and the computer processor is configured to determine the concentration of one or more components within a subject's bodily emission, based on the light that is detected in the ultraviolet range.

As described hereinabove, for some application, the computer processor is configured to determine the concentration of urinary creatinine, NT-titin, urinary pterin, urinary neopterin, urinary biopterin, fecal pterin, fecal neopterin, fecal biopterin, and/or prostaglandin E-major urinary metabolite, based upon the detected light.

For some such applications, the computer processor derives the concentration of creatinine by detecting an absorbance peak that is centered between 500 nm and 570 nm (e.g., between 510 nm and 550 nm). For some applications, the computer processor derives the concentration of creatinine based upon the absorbance at the above-described absorbance peak, relative to absorbance at other wavelengths. For some applications, the computer processor identifies an optical signature of creatinine within the visible light range, e.g., within a spectral range of between 350 nm and 700 nm. For some such applications, the computer processor detects a ratio between (a) the light intensity at a wavelength band within this range at which creatinine is expected to have an absorbance peak (e.g., between 500 nm and 570 nm, and or between 510 nm and 550 nm), and (b) the light intensity at a wavelength band within this range at which creatinine is expected to have an absorbance trough (e.g., between 600 and 680 nm, and/or between 360 nm and 440 nm).

For some such applications, the computer processor derives the concentration of a pterin and/or a pterin derivative (e.g., urinary pterin, urinary neopterin, urinary biopterin, and/or prostaglandin E-major urinary metabolite) by the light sensors detecting light within a spectral range of between 200 nm and 400 nm (e.g., within a spectral range of 240 and 360 nm). For some applications, the computer processor identifies an optical signature of a pterin and/or a pterin derivative (e.g., urinary pterin, urinary neopterin, urinary biopterin, and/or prostaglandin E-major urinary metabolite) within a spectral range of between 200 nm and 400 nm (e.g., within a spectral of 240 and 360 nm).

For some applications, the computer processor derives the concentration of a pterin and/or a pterin derivative (e.g., urinary pterin, urinary neopterin, urinary biopterin, and/or prostaglandin E-major urinary metabolite) by detecting an optical signature of the pterin and/or a pterin derivative in its acidic form within the detected light. For example, the computer processor may detect an absorbance trough that is centered between 240 and 265 nm (e.g., between 250 nm and 260 nm), an absorbance peak centered between 265 and 285 nm (e.g., between 270 nm and 280 nm), an absorbance trough that is centered between 285 and 310 nm (e.g., between 290 nm and 300 nm), and/or an absorbance peak centered between 330 and 360 nm (e.g., between 340 nm and 350 nm), in accordance with "New Results on the Photochemistry of Biopterin and Neopterin in Aqueous Solution," by Vignoni et al. (Photochemistry and Photobiology, 85: 365-373). For some applications, the computer processor derives the concentration of a pterin and/or a pterin derivative (e.g., urinary pterin, urinary neopterin, urinary biopterin, and/or prostaglandin E-major urinary metabolite) based upon the absorbance at the above-described absorbance peaks and/or troughs, relative to absorbance at other wavelengths. For some such applications, the computer processor detects a ratio between (a) the light intensity at a wavelength band at which the pterin or pterin derivative is expected to have an absorbance peak (e.g., as described above), and (b) the light intensity at a wavelength band at which the pterin or pterin derivative is expected to have an absorbance trough (e.g., as described above).

It is noted that the term "absorbance peak" should be interpreted as referring to a portion of the absorbance curve that exhibits a local maximum (e.g., a maximum within a range of plus or minus 25 nm from that portion of the curve). Similarly the term "absorbance trough" should be interpreted as referring to a portion of the absorbance curve that exhibits a local minimum (e.g., a minimum within a range of plus or minus 25 nm from that portion of the curve).

For some applications, the computer processor derives the concentration of a pterin and/or a pterin derivative (e.g., urinary pterin, urinary neopterin, urinary biopterin, and/or prostaglandin E-major urinary metabolite) by detecting an optical signature of the pterin and/or a pterin derivative in its basic form within the detected light. For example, the computer processor may detect an absorbance trough that is centered between 220 and 240 nm (e.g., between 225 nm and 235 nm), an absorbance peak centered between 245 and 265 nm (e.g., between 250 nm and 260 nm), an absorbance trough that is centered between 290 and 310 nm (e.g., between 295 nm and 305 nm), and/or an absorbance peak centered between 350 and 370 nm (e.g., between 355 nm and 365 nm), in accordance with "New Results on the Photochemistry of Biopterin and Neopterin in Aqueous Solution," by Vignoni et al. (Photochemistry and Photobiology, 85: 365-373). For some applications, the computer processor derives the concentration of a pterin and/or a pterin derivative (e.g., urinary pterin, urinary neopterin, urinary biopterin, and/or prostaglandin E-major urinary metabolite) based upon the absorbance at the above-described absorbance peaks and/or troughs, relative to absorbance at other wavelengths. For some such applications, the computer processor detects a ratio between (a) the light intensity at a wavelength band at which the pterin or pterin derivative is expected to have an absorbance peak (e.g., as described above), and (b) the light intensity at a wavelength band at which the pterin or pterin derivative is expected to have an absorbance trough (e.g., as described above).

For some applications, one or more probes (e.g., one or more biosynthetic probes) are added to the bodily emission (e.g., via a solution or a substrate that is dispensed into the toilet bowl) and are used to detect a pterin and/or a pterin derivative (e.g., urinary pterin, urinary neopterin, urinary biopterin, and/or prostaglandin E-major urinary metabolite) within the subject's bodily emission. For example, molecularly imprinted polymers, antibodies, and/or chemical molecules, that are configured to target pterins or pterin derivative (or a specific type of a pterin and/or a pterin derivative (e.g., urinary pterin, urinary neopterin, urinary biopterin, and/or prostaglandin E-major urinary metabolite)) are added to the bodily emission and the sensor module is configured to detect a parameter that is indicative of the concentration of the pterins and/or pterin derivatives by detecting a parameter of the probe.

For some applications, the probe is configured such that if the targeted pterins and/or pterin derivatives are present within the bodily emission, (a) the probes bond to the targeted pterins and/or pterin derivatives, (b) the pterins and/or pterin derivatives are thereby brought into reactive proximity with reporter molecules, and (c) the pterin or pterin derivatives react with the reporter molecule such that the reporter molecules undergo a change. For some applications, the reporter molecules undergo a color change, which is detected by the sensors. For some applications, the reporter molecule undergoes a change in a physical attribute, which may include a change in impedance, a change in the attenuation of sonic waves by the molecule, and/or a change in microwave activity in the presence of pterins or pterin derivatives. For some applications, one or more sensors are configured to detect such changes in the physical attribute of the reporter molecule. For some applications, the computer processor receives signals from the one or more sensors and analyzes the received signals.

For some applications, the concentration of myoglobin in a bodily emission is derived by detecting spectral bands that are centered around a wavelength that is in the range of 530 nm to 785 nm (e.g., between 530 nm and 600 nm). For some applications, two or more spectral bands are detected that are centered around approximately 540 nm, 565 nm, and 575 nm. In some applications, the widths of the spectral bands are greater than 3 nm (e.g., greater than 5 nm, or greater than 8 nm), and/or less than 40 nm (e.g., less than 20 nm, or less than 12 nm), e.g., between 3 and 40 nm, between 5 and 20 nm, or between 8 and 12 nm. For some applications, one or more ratios of the intensities of the aforementioned spectral bands with respect to one another are determined. For example, the ratio of the intensity of the spectral band that is centered around approximately 565 nm to that of the band centered around approximately 575 nm (or vice versa) may be determined, and/or the ratio of the intensity of the spectral band that is centered around approximately 565 nm to that of the band centered around approximately 540 nm (or vice versa) may be determined. For some applications, a different relationship between the intensities of the aforementioned spectral bands with respect to one another is determined.

For some applications, the one or more sensors 24 are configured to derive the concentration of NT-titin, by detecting light in the range of between 190 nm and 250 nm (e.g., between 200 nm and 240 nm). Within this range NT-titin has an optical signature including a peak at approximately 200 nm, a trough between 210 and 230 nm, and another peak at approximately 240 nm. For some applications, the computer processor determines derives the concentration of urinary NT-titin based upon the absorbance that exhibits that above-described optical signature, relative to absorbance at other wavelengths. For some applications, in response to determining that the subject has elevated urinary NT-titin (e.g., by detecting that the concentration of urinary NT-titin exceeds a predetermined threshold, such as a threshold of between 1.5 and 20 ng/ml), the computer processor determines that the subject's elevated creatinine is at least partially caused by catabolic processes (step 42b).

Urinary specific gravity measures the concentration of solutes in the urine, by measuring the ratio of urine density compared with water density. For some applications, the computer processor measures the subject's urinary specific gravity. Healthy adult urine will often have a specific gravity in the range of 1.010 to 1.030. For some applications, the sensor module is configured to generate a signal that is indicative of the absorption of light by the subject's urine, and the computer processor derives the specific gravity of the subject's urine based upon the signal. For some applications, the computer processor derives the specific gravity of the subject's urine based upon a sensor signal that is indicative of the absorption of cyan-green light (e.g., light within a wavelength band of 480-520 nm), by the subject's urine.

For some applications, the computer processor normalizes the concentration of an entity such as neopterin, biopterin, prostaglandin E-major urinary metabolite, a different pterin derivative, myoglobin, and/or NT-titin. For example, the concentration of one or more of these entities is normalized by dividing its urinary concentration by urinary creatinine concentration and/or urinary specific gravity. In some applications, urinary creatinine concentration and urinary specific gravity are determined as described hereinabove.

Applications of the disclosure described herein can take the form of a computer program product accessible from a computer-usable or computer-readable medium (e.g., a non-transitory computer-readable medium) providing program code for use by or in connection with a computer or any instruction execution system, such as a computer processor of user interface device 32, computer processor 28 disposed within housing 30, or a remote cloud-based computer processor. For the purpose of this description, a computer-usable or computer readable medium can be any apparatus that can include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. For some applications, the computer-usable or computer readable medium is a non-transitory computer-usable or computer readable medium.

Examples of a computer-readable medium include a semiconductor or solid-state memory, magnetic tape, a removable computer diskette, a random-access memory (RAM), a read-only memory (ROM), a rigid magnetic disk and an optical disk. Current examples of optical disks include compact disk-read only memory (CD-ROM), compact disk-read/write (CD-R/W) and DVD. For some applications, cloud storage is used.

A data processing system suitable for storing and/or executing program code will include at least one processor (e.g., a computer processor of user interface device 32, computer processor 28 disposed within housing 30, or a remote cloud-based computer processor) coupled directly or indirectly to memory elements (e.g., a memory of user interface device 32) through a system bus. The memory elements can include local memory employed during actual execution of the program code, bulk storage, and cache memories which provide temporary storage of at least some program code in order to reduce the number of times code must be retrieved from bulk storage during execution. The system can read the inventive instructions on the program storage devices and follow these instructions to execute the methodology of the embodiments of the disclosure.

Network adapters may be coupled to the processor to enable the processor to become coupled to other processors or remote printers or storage devices through intervening private or public networks. Modems, cable modem and Ethernet cards are just a few of the currently available types of network adapters.

Computer program code for carrying out operations of the present disclosure may be written in any combination of one or more programming languages, including an object-oriented programming language such as Java, Smalltalk, C++ or the like and conventional procedural programming languages, such as the C programming language or similar programming languages.

It will be understood that the algorithms described herein can be implemented by computer program instructions. These computer program instructions may be provided to a processor of a general purpose computer, special purpose computer, or other programmable data processing apparatus to produce a machine, such that the instructions, which execute via the processor of the computer (e.g., a computer processor of user interface device 32, computer processor 28 disposed within housing 30, or a remote cloud-based computer processor) or other programmable data processing apparatus, create means for implementing the functions/acts specified in the algorithms described in the present application. These computer program instructions may also be stored in a computer-readable medium (e.g., a non-transitory computer-readable medium) that can direct a computer or other programmable data processing apparatus to function in a particular manner, such that the instructions stored in the computer-readable medium produce an article of manufacture including instruction means which implement the function/act specified in the algorithms. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide processes for implementing the functions/acts specified in the algorithms described in the present application.

In some embodiments, the computer processors described herein are hardware devices programmed with computer program instructions to produce a special purpose computer. For example, when programmed to perform the algorithms described herein, the computer processor acts as a special purpose bodily-emission-analysis computer processor. For some applications, the operations described herein that are performed by computer processors transform the physical state of a memory, which is a real physical article, to have a different magnetic polarity, electrical charge, or the like depending on the technology of the memory that is used.

It will be appreciated by persons skilled in the art that the present disclosure is not limited to what has been particularly shown and described hereinabove. Rather, the scope of the present disclosure includes both combinations and subcombinations of the various features described hereinabove, as well as variations and modifications thereof that are not in the prior art, which would occur to persons skilled in the art upon reading the foregoing description.

## Claims

1. An apparatus for use with a bodily emission of the subject, the apparatus comprising:
one or more sensors (24) that are coupled to a toilet bowl, and that are configured to detect one or more parameters relating to the subject's bodily emission; and
at least one computer processor (28) configured to:
receive the one or more parameters relating to the subject's bodily emission from the one or more sensors,
derive a concentration of a pterin and/or a pterin derivative within the subject's bodily emission, and
determine a level of inflammation of the subject, at least partially in response thereto.

2. The apparatus according to claim 1, wherein the at least one computer processor (28) is configured to determine a level of systemic inflammation of the subject, at least partially in response to the concentration of the pterin and/or the pterin derivative within the subject's bodily emission.

3. The apparatus according to claim 1, wherein the at least one computer processor (28) is configured to monitor progression of chronic inflammatory disease of the subject, at least partially in response to the concentration of the pterin and/or the pterin derivative within the subject's bodily emission.

4. The apparatus according to claim 1, wherein the bodily emission includes urine and wherein the at least one computer processor (28) is configured to derive the concentration of the pterin and/or the pterin derivative within the subject's bodily emission by deriving a concentration of prostaglandin E-major urinary metabolite within the urine and/or by deriving a concentration of neopterin within the urine.

5. The apparatus according to claim 1, wherein the bodily emission includes feces and wherein the at least one computer processor (28) is configured to derive the concentration of the pterin and/or the pterin derivative within the subject's bodily emission by deriving a concentration of neopterin within the feces.

6. The apparatus according to any one of claims 1-5, wherein the one or more sensors (24) comprise one or more light sensors that are configured to detect light in a range of between 200 nm and 400 nm, and wherein the at least one computer processor (28) is configured to derive the concentration of the pterin and/or the pterin derivative within the subject's bodily emission by detecting an optical signature of the pterin and/or the pterin derivative within the detected light.

7. The apparatus according to claim 6, wherein the at least one computer processor (28) is configured to detect the optical signature of the pterin and/or the pterin derivative in its acidic form within the detected light by detecting light intensity that is indicative of one or more absorbance characteristics selected from the group consisting of:
an absorbance trough that is centered between 240 and 265 nm,
an absorbance peak centered between 265 and 285 nm,
an absorbance trough that is centered between 285 and 310 nm, and
an absorbance peak centered between 330 and 360 nm.

8. The apparatus according to claim 7, wherein the at least one computer processor (28) is configured to detect the optical signature of the pterin and/or the pterin derivative in its acidic form within the detected light by detecting a ratio between light intensity at at least one of one of the absorbance troughs and light intensity at at least one of the absorbance peaks.

9. The apparatus according to claim 6, wherein the at least one computer processor (28) is configured to detect the optical signature of the pterin and/or the pterin derivative in its basic form within the detected light by detecting light intensity that is indicative of one or more absorbance characteristics selected from the group consisting of:
an absorbance trough that is centered between 220 and 240 nm,
an absorbance peak centered between 245 and 265 nm,
an absorbance trough that is centered between 290 and 310 nm, and
an absorbance peak centered between 350 and 370 nm.

10. The apparatus according to claim 9, wherein the at least one computer processor (28) is configured to detect the optical signature of the pterin and/or the pterin derivative in its basic form within the detected light by detecting a ratio between light intensity at at least one of one of the absorbance troughs and light intensity at at least one of the absorbance peaks.

11. A method for use with a bodily emission of the subject, the method comprising:
using at least one computer processor (28):
receiving the one or more parameters relating to the subject's bodily emission from one or more light sensors (24) that are coupled to a toilet bowl,
deriving a concentration of a pterin and/or a pterin derivative within the subject's bodily emission, and
determining a level of inflammation of the subject, at least partially in response thereto.

12. The method according to claim 11, wherein determining the level of inflammation of the subject at least partially in response in response to the concentration of the pterin and/or the pterin derivative within the subject's bodily emission comprises determining a level of systemic inflammation of the subject, at least partially in response to the concentration of the pterin and/or the pterin derivative within the subject's bodily emission.

13. The method according to claim 11, wherein determining the level of inflammation of the subject at least partially in response in response to the concentration of the pterin and/or the pterin derivative within the subject's bodily emission comprises monitoring progression of chronic inflammatory disease of the subject, at least partially in response to the concentration of the pterin and/or the pterin derivative within the subject's bodily emission.

14. The method according to any one of claims 11-13, wherein the one or more light sensors (24) are configured to detect light in a range of between 200 nm and 400 nm, and wherein deriving the concentration of the pterin and/or the pterin derivative within the subject's bodily emission comprises detecting an optical signature of the pterin and/or the pterin derivative within the detected light.

15. The method according to claim 14, wherein detecting the optical signature of the pterin and/or the pterin derivative within the detected light comprises:
(a) detecting the optical signature of the pterin and/or the pterin derivative in its acidic form within the detected light by detecting light intensity that is indicative of one or more absorbance characteristics selected from the group consisting of:
an absorbance trough that is centered between 240 and 265 nm,
an absorbance peak centered between 265 and 285 nm,
an absorbance trough that is centered between 285 and 310 nm, and
an absorbance peak centered between 330 and 360 nm; and/or
(b) detecting the optical signature of the pterin and/or the pterin derivative in its basic form within the detected light by detecting light intensity that is indicative of one or more absorbance characteristics selected from the group consisting of:
an absorbance trough that is centered between 220 and 240 nm,
an absorbance peak centered between 245 and 265 nm,
an absorbance trough that is centered between 290 and 310 nm, and
an absorbance peak centered between 350 and 370 nm.
